(19) Europäisches Patentamt

European Patent Office

Office européen des brevets

(11) Numéro de publication : **0 270 641 B1**

(12) **FASCICULE DE BREVET EUROPEEN**

(45) Date de publication du fascicule du brevet :
18.09.91 Bulletin 91/38

(51) Int. Cl.⁵ : **A61F 5/01**

(21) Numéro de dépôt : **87904055.8**

(22) Date de dépôt : **19.06.87**

(86) Numéro de dépôt international :
**PCT/FR87/00235**

(87) Numéro de publication internationale :
**WO 87/07828 30.12.87 Gazette 87/29**

(54) **APPAREIL ORTHOPEDIQUE POUR GENOU INSTABLE.**

(30) Priorité : **20.06.86 FR 8609062**

(43) Date de publication de la demande :
**15.06.88 Bulletin 88/24**

(45) Mention de la délivrance du brevet :
**18.09.91 Bulletin 91/38**

(84) Etats contractants désignés :
**AT BE CH DE GB LI NL SE**

(56) Documents cités :
**DE-A- 2 317 561
DE-A- 3 440 521
FR-A- 2 350 091
FR-A- 2 441 382
FR-A- 2 477 409
US-A- 2 460 895**

(73) Titulaire : **CADORET, Alain J-B.
71, rue de Starnberg
F-35800 Dinard (FR)**

(72) Inventeur : **CADORET, Alain J-B.
71, rue de Starnberg
F-35800 Dinard (FR)**

(74) Mandataire : **Le Guen, Louis François
Cabinet Louis Le Guen 38, rue Levavasseur
B.P. 91
F-35802 Dinard Cédex (FR)**

# Description

La présente invention concerne un appareil orthopédique pour genou instable à la suite de lésions ligamentaires, articulaires, neurologiques ou musculaires.

Les laxités chroniques du genou sont handicapantes d'une manière générale, et plus particulièrement pour certaines activités professionnelles ou sportives.

Par la rééducation, on peut pallier la déficience des ligaments en faisant travailler de manière intensive et contrôlée les muscles périarticulaires. On permet également aux capteurs proprioceptifs capsulaires, ligamentaires, musculaires de répondre immédiatement à tout déplacement articulaire dangereux afin qu'une contraction réflexe des muscles périarticulaires arrête ce déplacement.

Une rééducation bien faite permet une bonne récupération fonctionnelle, même dans les cas où les mouvements anormaux objectivés par l'examen clinique sont importants. Toutefois, la rééducation a ses limites qui sont vite atteintes dans certaines activités telles que le sport de haut niveau.

En présence d'un genou instable chronique que la chirurgie et la rééducation ne peuvent améliorer davantage, il reste alors la solution de réduire son activité physique en fonction des possibilités fonctionnelles restantes ou celle de prévoir une orthèse palliant le déficit capsulo-ligamentaire en gênant le moins possible la fonction normale du genou.

De telles orthèses existent, parmi lesquelles la simple genouillère en tissu élastique, le bandage type "strapping" ou des dispositifs à articulation à axe monocentrique. Bien que satisfaisant, le strapping a une efficacité trop faible en face d'une sollicitation sérieuse. Quant aux dispositifs à articulation à axe monocentrique, ils sont peu prescrits car, du fait que leur axe ne coïncide pas exactement avec celui du genou, ils ont tendance à glisser sur le membre inférieur au cours des flexions répétées et nécessitent donc une fixation aux chaussures, par tourillon, ou à la ceinture, ce qui est très contraignant.

Le mouvement du genou est en effet très complexe. A titre indicatif, au cours d'une flexion, le déplacement d'un point de la face antéro-interne de la tubérosité tibiale par rapport à un point du condyle interne se décompose en une rotation autour de l'axe de flexion-extension du genou, une translation dans le plan antéro-postérieur correspondant à l'avancée du plateau tibial sous les condyles, une translation correspondant à l'inégalité des rayons de courbure des surfaces en contact lors du mouvement et un petit déplacement de varus.

Il a été imaginé des systèmes d'articulation reproduisant plusieurs composantes du mouvement du genou. Un tel système est décrit dans le document FR-A-2 477 409. L'articulation est monocentrique mais une partie du support extérieur est souple pour permettre une certaine rotation de la jambe par rapport à la cuisse.

Dans le document FR-A-2 546 743, l'articulation reproduit le mouvement de roulement et de glissement du genou.

Bien entendu, ces orthèses ne reproduisent qu'en partie le mouvement du genou et ne permettent donc pas des mouvements dans tous les secteurs physiologiques du genou. Celui-ci est de toute façon beaucoup trop complexe pour que l'axe d'un système mécanique simple coïncide exactement avec celui du genou tout au long de son action lors d'une flexion ou d'une extension.

Dans la demande de brevet français FR-A-2 441 382, le présent demandeur a imaginé un système articulé qui se laisse guider passivement par le genou lui-même, sauf dans les secteurs non physiologiques, ce qui évitait de calculer les centres articulaires. L'orthèse se compose d'un système de contention fixé sur la cuisse et la jambe et d'un système d'articulation en regard des deux faces du genou. Par rapport à l'élément de jambe du système de contention, l'articulation est libre de certains mouvements n'affectant pas l'efficacité de l'orthèse. L'orthèse respecte bien la physiologie du genou. Elle est légère et confortable. Elle est cependant un peu encombrante et ne correspond pas à tous les besoins. En particulier, elle ne comporte pas de réglage du valgus-varus.

Or, un tel réglage présente des intérêts multiples. Il permet d'adapter efficacement l'orthèse à chaque morphologie en positionnant son axe de façon très précise. Il permet de régler la tension du valgus ou du varus ou de lutter contre la déformation en valgus ou en varus, que la cause de cette déformation soit rhumatismale, neurologique ou musculaire. En phase post-chirurgicale, il constitue un moyen de restreindre les mouvements de latéralité pendant la période de cicatrisation des ligaments opérés sans obligation d'immobiliser le genou. De plus, il permet la décharge articulaire, côté interne ou côté externe, pour limiter l'hyperpression uni-compartimentale d'origine arthrosique par défaut d'axe.

Il peut être également intéressant de limiter la flexion-extension, notamment si l'ortèse est utilisée en phase post-chirurgicale. Si on limite, par exemple, l'extension à -20° et la flexion à 60°, on évite la tension des sutures ligamentaires, tout en permettant une certaine mobilité du genou.

Un objet de la présente invention est donc de prévoir une orthèse de genou comportant des réglages du valgus-varus et, éventuellement, de la flexion-extension.

Un autre objet de l'invention consiste à rechercher un système mécanique simple, robuste et le moins encombrant possible.

Un autre objet, encore, de l'invention consiste à améliorer le dispositif de contention de la jambe et de la cuisse.

Selon l'invention, l'orthèse comprend un élément de contention de cuisse comportant un montant interne et un montant externe reliés par une embrasse, un élément de contention de jambe comportant également un montant interne et un montant externe reliés par au moins une embrasse, les montants internes et les montants externes étant respectivement reliés par deux articulations latérales, connu par FR-A-2 441 382, caractérisé en ce que l'articulation externe comportant deux pattes articulées en genouillère pour bloquer l'extension vers l'avant, le longueur d'une des pattes étant règlable, et en ce que l'articulation interne comportant une patte supérieure articulée autour d'un axe sensiblement transversal, selon un montage en genouillère, avec la partie supérieure d'un croisillon dont la partie inférieure est articulée à l'extrémité d'une biellette dont l'autre extrémité est articulée à la patte inférieure de ladite articulation interne selon un axe antéro-postérieur. La longueur de la biellette est règlable.

Selon une éxécution de l'invention, les articulations sont des articulations à chape.

Selon une autre éxécution de l'invention, au moins l'une des pattes de l'articulation externe et ladite biellette de l'articulation interne sont formées de deux parties dont l'une porte une tige filetée engagée dans une extrémité taraudée de l'autre partie, pour permettre de régler leur longueur.

Selon une autre éxécution de l'invention, sur les tiges filetées de ladite patte et de ladite biellette, sont montés des contre-écrous limitant ou bloquant la rotation d'une de leur partie par rapport à leur autre partie.

Selon une autre éxécution de l'invention, les embrasses de l'élément de contention de cuisse et de l'élément de contention de jambe sont articulées dans leur partie médiane, pour s'adapter aux variations de volumes musculaires et aux déplacements du tibia et du péroné pendant les différents mouvements.

Selon une autre éxécution de l'invention, l'élément de contention de jambe comporte deux embrasses.

Selon une autre éxécution de l'invention, l'élément de contention de cuisse comporte des barres de triangulation entre son embrasse et ses montants.

Selon une autre éxécution de l'invention, aux faces internes des embrasses de jambe sont fixées des surfaces d'appui en matériau souple.

Selon une autre éxécution de l'invention, lesdites surfaces d'appui sont obtenues par moulage, directement sur la jambe du sujet.

Selon une autre éxécution de l'invention, la surface d'appui inférieure s'accroche sur la partie supérieure des malléoles.

Selon une autre éxécution de l'invention, les articulations comportent des moyens limitant l'extension et/ou la flexion de l'orthèse.

Pour augmenter encore le confort et l'efficacité, de l'orthèse, on peut prévoir, entre l'armature de l'élément de contention de cuisse et la cuisse elle-même, des organes de fixation qui, d'une part, ont une surface de contact importante avec la cuisse et, d'autre part, permettent à l'armature dudit élément de contention de cuisse une certaine liberté de mouvement sensiblement dans l'axe de la cuisse.

Selon une autre éxécution de l'invention, l'élément de contention de cuisse comporte un organe de fixation supérieur comprenant une bande avant, pourvue d'une garniture souple, de largeur supérieure à l'embrasse, fixée aux extrémités de ladite embrasse, la fixation d'extrémité, côté interne, permettant un léger déplacement longitudinal de l'embrasse par rapport à la cuisse, et un organe de fixation inférieur comprenant une bande avant, pourvue d'une garniture souple, dont la partie, côté interne de la cuisse, comporte un manchon d'axe sensiblement parallèle à celui de la cuisse, dans lequel passe le montant interne de l'élément de contention de cuisse, l'ouverture dudit manchon ayant une largeur supérieure à celle dudit montant, de telle sorte que celui-ci puisse s'y déplacer légèrement vers l'avant ou vers l'arrière, ainsi que longitudinalement par rapport à la cuisse, ladite bande avant passant, de l'autre côté, dans une boucle fixée sur la face extérieure du montant externe, lesdites bandes avant desdits organes supérieur et inférieur de fixation étant prolongées, à leurs extrémités, par des sangles munies de moyen de serrage et d'attache.

Selon une autre éxécution de l'invention, ladite bande avant de l'organe de fixation supérieur comporte dans sa partie médiane un manchon dans laquelle passe ladite embrasse, la largeur de l'ouverture dudit manchon étant supérieure à celle de ladite embrasse, pour que cette dernière puisse légèrement s'y déplacer longitudinalement par rapport à la cuisse.

Selon une autre éxécution de l'invention, les sangles des organes de fixation sont élastiques.

Selon une autre éxécution de l'invention, la bande avant de l'organe de fixation inférieur comporte une échancrure de dégagement de la rotule.

L'invention et l'éxécutions de l'invention mentionnées ci-dessus, ainsi que d'autres, apparaîtront plus clairement à la lecture de la description suivante d'un exemple de réalisation, ladite description étant faite en relation avec les dessins joints, parmi lesquels:

la Fig. 1 est une vue en perspective d'une orthèse selon l'invention en position fléchie,

la Fig. 2 est une vue en perspective, à plus petite échelle, de l'orthèse de la Fig. 1 en extension,

la Fig. 3 est une vue agrandie, en perspective, de l'articulation interne de l'orthèse, et

la Fig. 4 est une vue de l'élément de contention de cuisse pourvu d'organes de fixation spécialement conçus.

L'orthèse de l'invention comporte un élément supérieur de contention de cuisse 1, un élément inférieur de contention de jambe 2 et un dispositif d'articulation 3 entre les deux éléments de contention.

L'élément de contention de cuisse 1 est formé de deux montants latéraux 4 et 5 et d'une embrasse antérieure 6 soudée par ses extrémités aux extrémités supérieures des montants. L'embrasse 6 comporte une articulation autour d'un axe X-X dans sa partie médiane permettant d'écarter plus ou moins les montants 4 et 5. Par contre, l'articulation ne permet pas de mouvement de translation des montants 4 et 5 l'un par rapport à l'autre.

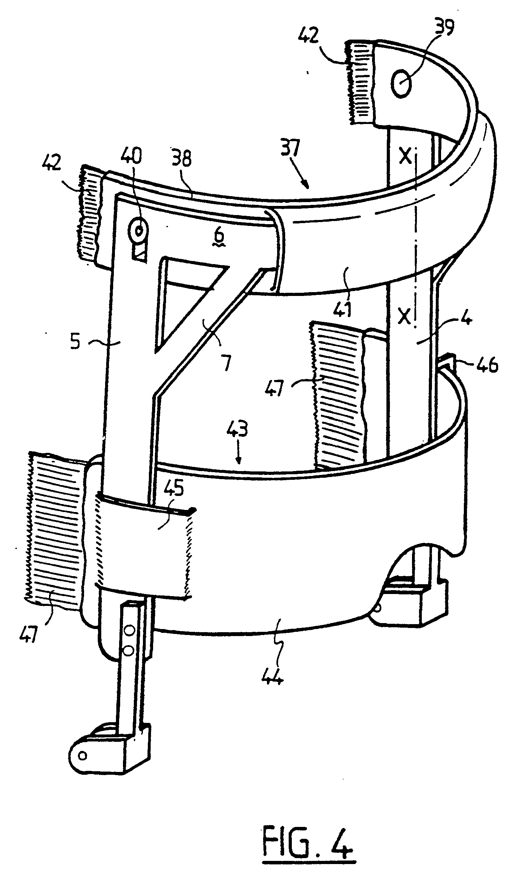

Une barre de triangulation 7 est prévue entre chaque montant 4 ou 5 et l'embrasse 6 pour donner plus de solidité à l'ensemble. Le long de chaque montant, sont fixées deux boucles 8 pour le passage de sangles. La sangle supérieure, au niveau de l'embrasse 6, est large et légèrement élastique. La sangle inférieure, plus étroite, passe en arrière de la cuisse, 2 à 3 cm au-dessus du creux poplité. Dans une forme préférée de l'invention, on prévoit que l'embrasse 6 soit appliquée au niveau du deuxième tiers supérieur de la cuisse. Des organes de fixation plus élaborés que des simples sangles peuvent être prévus, qui seront décrits plus en détail dans la suite, en relation avec la Fig. 4.

Près de leur extrémité inférieure, les montants 4 et 5 comportent deux rangées longitudinales de trous équidistants 9. Les trous 9 permettent le rivetage des montants 4 et 5 sur le dispositif d'articulation 3. Ils offrent la possibilité de régler la longueur des montants 4 et 5 sur quelques centimètres.

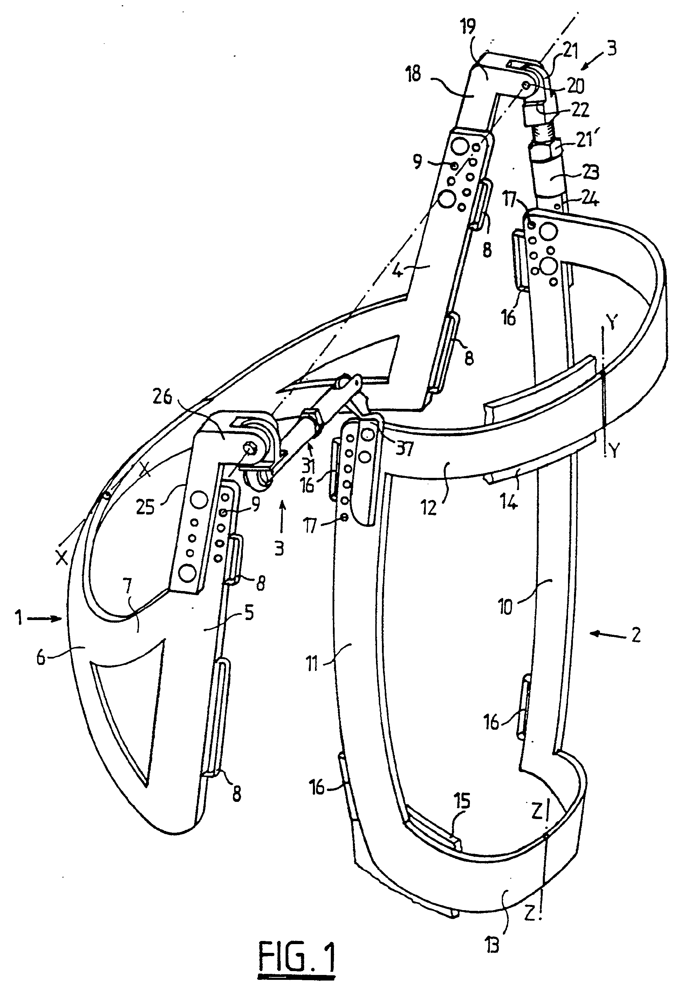

L'élément de contention de jambe 2 est formé de deux montants latéraux 10 et 11 réunis par deux embrasses antérieures 12 et 13 articulées, comme l'embrasse 6, dans leur partie médiane autour d'axes Y-Y et Z-Z, respectivement. L'embrasse supérieure 12 s'applique à hauteur de l'épine tibiale antérieure. Sur sa face interne, est fixée une surface d'appui 14, partiellement représentée à la Fig. 1, en matériau souple et de largeur suffisante pour s'accrocher de façon confortable sur la partie supérieure du relief musculaire de jambe formé des péroniers latéraux et des jumeaux. La surface d'appui 14 peut être réalisée en un matériau approprié qu'on utilise en orthopédie. Elle peut notamment être moulée directement sur le sujet.

L'embrasse inférieure 13 se place au niveau susmalléolaire. Y est fixée une surface d'appui 15, également représentée partiellement à la Fig. 1, éventuellement moulée, qui permet l'accrochage sur la partie supérieure des malléoles.

Chaque montant 10 ou 11 comporte deux boucles 16 au niveau des embrasses 12 et 13. Dans les boucles 16, passent deux sangles assurant le maintien de l'élément 2 sur le membre. Un problème des orthèses de jambe est d'empêcher leur descente au cours des mouvements. L'élément de jambe supportant l'élément de cuisse, il faut qu'il ne bouge pas. La solution de l'appui sur les deux malléoles est satisfaisante. Pour améliorer encore la fixation de l'élément de contention de jambe 2, on peut prévoir de disposer les sangles obliquement en les faisant passer d'un côté dans une boucle supérieure et, de l'autre côté, dans une boucle inférieure.

Les extrémités supérieures des montants 10 et 11, comme les extrémités inférieures des montants 4 et 5, comportent deux rangées longitudinales de trous équidistants 17 permettant le rivetage des montants 10 et 11 sur le dispositif d'articulation 3 avec plusieurs possibilités de réglage.

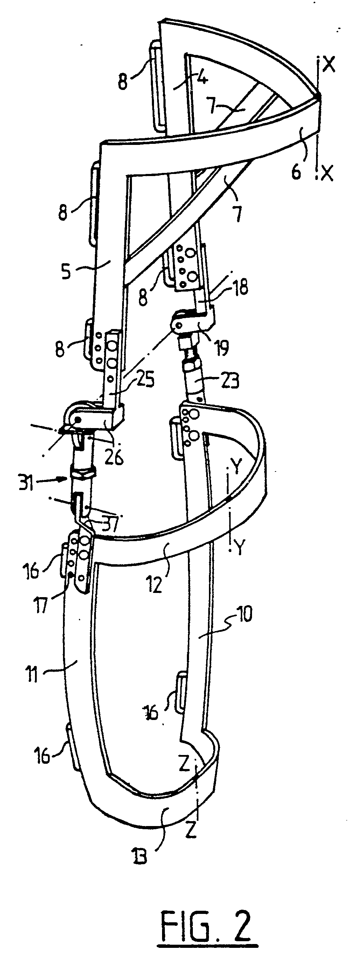

Le montant externe 4 de l'élément de contention supérieur 1 est fixé par son extrémité inférieure à une patte 18 du dispositif d'articulation 3, au moyen de rivets passant dans certains des trous 9 choisis en fonction de la morphologie du sujet. La patte 18 prolonge le montant 4. A son extrémité inférieure, est soudée perpendiculairement une chape 19 orientée vers l'arrière Fig. 2. La chape 19 porte un axe d'articulation transversal horizontal 20. En pratique, la patte 18 et la chape 19 sont, de préférence, en une seule pièce.

Entre les deux bras de la chape 19, est articulée autour de l'axe 20 une patte 21 dirigée vers le bas. Juste au-dessous de la chape 19, la patte 21 est épaissie de manière à présenter des épaulements 22. En butant sous les bras de la chape 19, les épaulements 22 limitent le pivotement de la patte 21 vers l'avant, dans le sens de l'hyper-extension, Fig. 2. La patte 21 est prolongée vers le bas par une tige filetée engagée dans une pièce cylindrique taraudée 23 dont l'autre extrémité est soudée à une patte 24 fixée au montant 10 de l'élément inférieur 2 au moyen de rivets. Cette liaison entre la patte 21 et la patte 24 permet entre elles un léger jeu de rotation accompagnant la rotation automatique du genou. Avec un contreécrou 21' sur la tige filetée de la patte 21, on peut bloquer ou limiter ladite rotation automatique du genou. Le système de genouillère formé par les épaulements 22 et les bords inférieurs des bras de la chape 19 limite l'hyper-extension.

La fabrication de cette partie du dispositif d'articulation 3 doit répondre à deux exigences essentielles, de rigidité et de résistance à l'usure, car le moindre jeu entraînerait une certaine inefficacité de l'orthèse.

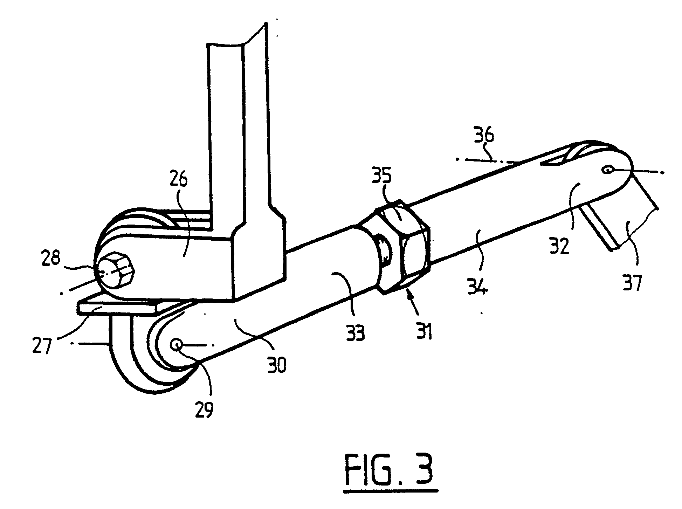

De même que le montant externe 4, le montant interne 5 est prolongé vers le bas par une patte 25 dont l'extrémité inférieure porte une chape 26 orientée horizontalement vers l'arrière, Fig. 2, sensiblement au même niveau que la chape 19. Dans la chape 26, comme on le voit plus clairement à la Fig. 3, est articulée la partie supérieure d'un croisillon 27 autour d'un axe transversal 28. La partie médiane du croisillon 27 forme une butée vers l'avant pour les bords inférieurs de la chape 26. Le système de genouillère ainsi formé limite l'hyper-extension côté interne. La partie inférieure du croisillon 27 est articulée autour d'un axe 29 perpendiculaire à l'axe 28, porté par une chape 30. La chape 30 se trouve à l'extrémité d'une biellette 31 dont l'autre extrémité comporte également une chape 32 dont la fente est sensiblement parallèle à celle de la chape 30. Le croisillon 27 forme donc avec la patte 26 et la biellette 31 un montage à la Cardan. La biellette 31 comporte une partie 33 prolongée par une tige filetée et une partie taraudée 34 pour recevoir ladite tige filetée. La partie 33 et la partie 34 ont donc entre elles un léger jeu de rotation, comme les pattes 21 et 24. Ce léger jeu accompagne, côté interne, la rotation automatique du genou. Un contre-écrou 35 engagé sur la tige filetée de la partie 33 permet de bloquer ou limiter, côté interne, la rotation automatique du genou. Bien entendu, les réglages du contre-écrou 21' et du contre-écrou 35 sont complémentaires.

Côté externe, après avoir démonté l'axe 20, on peut faire varier la longueur de l'ensemble formé par les pattes 21 et 23 en vissant plus ou moins la tige filetée prolongeant la patte 21 dans la patte 23. De même, côté interne, après avoir démonté l'axe 28, on peut régler la longueur de la biellette 31. Une fois les axes 20 et 28 remontés, les longueurs de l'ensemble des pattes 21 et 23, d'une part, et de la biellette 31, d'autre part, sont définies, au léger jeu de rotation près. Ces deux réglages de longueur combinés permettent d'effectuer des corrections de valgus-varus. Ils permettent également de faire correspondre précisément l'axe de l'orthèse avec l'axe articulaire. On pourrait également opérer ces réglages de longueur en choisissant la biellette 31 et la patte 21-23 dans des jeux de pièces de longueurs différentes mais fixes. La rotation pourrait être assurée en fabriquant lesdites pièces dans un matériau ayant des caractéristiques de torsion appropriées.

Dans la chape 32, autour d'un axe 36 sensiblement parallèle à l'axe 29, est articulée l'extrémité supérieure d'une patte 37 dont l'extrémité inférieure, après torsion à 90°, est rivetée au montant 11.

Le système d'articulation peut comporter, en outre, des moyens, non représentés, pour limiter l'extension et/ou la flexion de l'orthèse.

De plus, l'élément de contention de cuisse 1 comportera avantageusement des organes de fixation spécialement étudiés pour le confort et l'efficacité de l'orthèse. De tels organes de fixation sont montrés à la Fig. 4.

L'organe de fixation supérieur 37 comporte une bande avant 38 appliquée sur la face interne de l'embrasse 6. La bande 38 est sensiblement plus large que l'embrasse 6 et elle comporte, côté cuisse, une garniture souple appropriée, en polyéthylène par exemple. La bande 38 est fixée en 39 à l'extrémité de l'embrasse 6 adjacente au montant externe 4 et en 40 à l'autre extrémité de l'embrasse, adjacente au montant interne 5. En 40, la fixation permet un léger jeu, dans l'axe de la cuisse, entre l'embrasse 6 et la bande 38. De préférence, la bande 38 comporte un manchon 41 dans sa partie médiane, dans lequel passe la partie médiane de l'embrasse 6. L'ouverture du manchon 41 a une largeur supérieure à celle de la bande 38, de manière à permettre le mouvement relatif entre l'embrasse et la bande, mentionné précédemment. A ses extrémités, la bande 38 est prolongée par des sangles 42 pourvues de moyens de serrage et d'attache classiques. De préférence, les sangles 42 ont une certaine élasticité pour des raisons de confort et les moyens de serrage et de fixation sont du type Velcro.

L'organe de fixation inférieur 43 comporte une bande large 44 munie côté cuisse, comme la bande 38, d'une garniture souple. Côté interne, la bande 44 comporte sur sa face extérieure un manchon 45 dont l'axe est sensiblement parallèle à celui de la cuisse. Dans l'ouverture du manchon 45, passe le montant interne 5. L'ouverture du manchon 45 est sensiblement plus large que le montant 5, de manière à ce qu'il puisse s'y déplacer vers l'avant ou vers l'arrière, ainsi que longitudinalement par rapport à la cuisse. Par contre, il n'y a pas de jeu du montant 5 dans le manchon 45 dans la direction radiale.

Côté externe, le montant 4 comporte une boucle 46 sur sa face extérieure, dans laquelle passe la bande 44. Comme la bande 38, la bande 44 est prolongée par des sangles 47 pourvues de moyens de serrage et d'attache. De préférence, la partie inférieure de la bande 44 comporte une échancrure dans sa partie médiane pour dégager la rotule.

De tels organes de fixation améliorent sensiblement le confort de l'orthèse. De plus, ils permettent de maintenir le montant externe bien en place par rapport à la cuisse alors que le montant 5 peut se déplacer légèrement dans des secteurs autorisés bien déterminés.

Le système d'articulation permet à l'orthèse de s'adapter parfaitement aux variations de volume musculaire des muscles périarticulaires du genou, notamment lors de la flexion incomplète qui a tendance à écarter les articulations externe et interne. Elle laisse également une grande liberté de mouvement, comme s'asseoir en tailleur, par exemple, d'où son intérêt pour la reprise sportive.

L'orthèse de l'invention peut être utilisée dans pratiquement tous les cas d'instabilité du genou. Ses divers réglages et la conception des éléments de contention de cuisse et de jambe permettent de l'adapter au sujet dans des conditions optimales de tolérance et de confort. Son dispositif d'articulation est simple et donc fiable. Il est également peu encombrant.

Les applications de l'orthèse sont également diverses: empêcher les mouvements anormaux du genou, effectuer des corrections en valgus ou en varus, traiter des arthroses du genou, soulager des régions malades en les déchargeant, rééduquer les muscles propriocepteurs à la sortie d'un plâtre, permettre une mobilité contrôlée et protégée du genou après chirurgie ligamentaire du genou, etc.

## Revendications

1. Appareil orthopédique pour genou instable à la suite de lésions ligamentaires, articulaires, neurologiques ou musculaires, comprenant un élément de contention de cuisse (1) formé d'un montant externe (4) et d'un montant interne (5) reliés par une embrasse (6), un élément de contention de jambe (2) formé également d'un montant externe (10) et d'un montant interne (11) reliés par au moins une embrasse, les montants externes (4, 10) et les montants internes (5, 11) étant respectivement reliés par deux articulations latérales, caractérisé en ce que l'articulation externe comporte deux pattes (18 et 21-23-24) articulées en genouillère pour bloquer l'extension en avant, la longueur d'une des pattes (18;21-23-24) étant réglable, et en ce que l'articulation interne comporte une patte supérieure (25) articulée autour d'un axe sensiblement transversal, selon un montage en genouillère, avec la partie supérieure d'un croisillon (27) dont la partie inférieure est articulée à l'extrémité d'une biellette (31) dont l'autre extrémité est articulée à une patte inférieure (37) de ladite articulation interne selon un axe antéro-postérieur, la longueur de la biellette (31) étant réglable.

2. Appareil orthopédique selon la revendication 1, caractérisé en ce que les articulations sont des articulations à chape.

3. Appareil orthopédique selon la revendication 1 ou 2, caractérisé en ce que la patte de longueur réglable (21-23-24) de l'articulation externe et la biellette (31) de l'articulation interne sont formées de deux parties (33 et 34 ou 21 et 23-24) dont l'une porte une tige filetée engagée dans une extrémité taraudée de l'autre partie.

4. Appareil orthopédique selon l'une des revendications 1 à 3, caractérisé en ce que sur les tiges filetées de la patte (21-23-24) et de la biellette (31), sont montés des contre-écrous (21', 35) limitant ou bloquant la rotation d'une de leur partie par rapport à leur autre partie.

5. Appareil orthopédique selon l'une des revendications 1 à 4, caractérisé en ce que les embrasses de l'élément de contention de cuisse (1) et de l'élément de contention de jambe (2) sont articulées dans leur partie médiane.

6. Appareil orthopédique selon l'une des revendications 1 à 5, caractérisé en ce que l'élément de contention de jambe (2) comporte deux embrasses (12, 13).

7. Appareil orthopédique selon l'une des revendications 1 à 6, caractérisé en ce que l'élément de contention de cuisse (1) comporte des barres de triangulation (7) entre son embrasse (6) et ses montants (4, 5).

8. Appareil orthopédique selon l'une des revendications 1 à 7, caractérisé en ce qu'aux faces internes des embrasses de jambe (12, 13), sont fixées des surfaces d'appui en matériau souple (14, 15).

9. Appareil orthopédique selon la revendication 8, caractérisé en ce que les surfaces d'appui (14, 15) sont obtenues par moulage, sur la jambe du sujet.

10. Appareil orthopédique selon la revendication 8 ou 9, caractérisé en ce que la surface d'appui inférieure (15) s'accroche sur la partie supérieure des malléoles.

11. Appareil orthopédique selon l'une des revendications 1 à 10, caractérisé en ce qu'il comporte des moyens pour en limiter l'extension et/ou la flexion.

12. Appareil orthopédique selon l'une des revendications 1 à 11, caractérisé en ce que l'élément de contention de cuisse (1) comporte un organe de fixation supérieur (37) comprenant une bande avant (38), pourvue d'une garniture souple, de largeur supérieure à l'embrasse (6), fixée aux extrémités de ladite embrasse (6), la fixation d'extrémité (40), côté interne, permettant un léger déplacement longitudinal de l'embrasse (6) par rapport à la cuisse, et un organe de fixation inférieur comprenant une bande avant (44), pourvue d'une garniture souple, dont la partie, côté interne de la cuisse, comporte un manchon (45) d'axe sensiblement parallèle à celui de la cuisse, dans lequel passe le montant interne (5) de l'élément de contention de cuisse (1), l'ouverture du manchon (45) ayant une largeur supérieure à celle du montant (5), de telle sorte que celui-ci puisse s'y déplacer légèrement vers l'avant ou vers l'arrière, ainsi que longitudinalement par rapport à la cuisse, la bande (44) passant, de l'autre côté, dans une boucle 46 fixée sur la face extérieure du montant externe (4), lesdites bandes 38 et 44 étant prolongées, à leurs extrémités, par des sangles (42, 47) munies de moyens de serrage et d'attache.

13. Appareil orthopédique selon la revendication 12, caractérisé en ce que la bande avant (38) de l'organe de fixation supérieur (37) comporte dans sa partie médiane un manchon (41) dans laquelle passe l'embrasse (6), la largeur de l'ouverture du manchon (41) étant supérieure à celle de l'embrasse (6), pour que cette dernière puisse légèrement s'y déplacer longitudinalement par rapport à la cuisse.

14. Appareil orthopédique selon la revendication 12 ou 13, caractérisé en ce que les sangles (42, 47) des organes de fixation (37, 43) ont une certaine élasticité en vue d'améliorer le confort.

15. Appareil orthopédique selon l'une des revendications 12 à 14, caractérisé en ce que la bande avant (44) de l'organe de fixation inférieur (43) comporte une échancrure de dégagement de la rotule.

## Claims

1. Orthopaedic apparatus for unstable knee following ligamentary, articular, neurological or muscular injuries comprising a thigh retention element (1) formed by an outer upright (4) and an inner upright (5) connected by a loop (6), a leg retention element (2) likewise formed by an outer upright (10) and an inner upright (11) connected by means of a loop, the outer uprights (4, 10) and the inner uprights (5, 11) being respectively connected by two lateral articulations, characterised in that the outer articulation comprises two legs (18 and 21-23-24), knuckle-jointed to lock the extension forward, the length of one of the legs (18; 21-23-24) being adjustable and in that the inner articulation comprises an upper leg (25) articulated around a substantially transverse axis according to a knuckle-jointed mounting with the upper part of a cross-piece (27) the lower part of which is articulated by the end of a rod (31) the other end of which is articulated to a lower leg (37) of the said inner articulation according to an antero-posterior axis the length of the rod (31) being adjustable.

2. Orthopaedic apparatus according to claim 1, characterised in that the articulations are fork-link articulations.

3. Orthopaedic apparatus according to claim 1 or 2, characterised in that the leg of adjustable length (21-23-24) of the outer articulation and the rod (31) of the inner articulation are formed by two parts (33 and 34 or 21 and 23-24) one of which carries a screw-threaded rod engaged in a threaded end of the other part.

4. Orthopaedic apparatus according to one of the claims 1 to 3, characterised in that on the threaded rods of the leg (21-23-24) and of the rod (31) are mounted lock-nuts (21$^1$, 35) limiting or locking the rotation of one of their parts in respect of their other part.

5. Orthopaedic apparatus according to one of the claims 1 to 4, characterised in that the loops of the thigh retention element (1) and of the leg retention element (2) are articulated at their middle part.

6. Orthopaedic apparatus according to one of the claims 1 to 5, characterised in that the leg retention part (2) comprises two loops (12, 13).

7. Orthopaedic apparatus according to one of the claims 1 to 6, characterised in that the thigh retention element (1) comprises triangulation bars (7) between its loop (6) and its uprights (4, 5).

8. Orthopaedic apparatus according to one of the claims 1 to 7, characterised in that to the inner faces of the leg loops (12, 13) are fixed supporting surfaces made of flexible material (14, 15).

9. Orthopaedic apparatus according to claim 8, characterised in that the supporting surfaces (14, 15) are obtained by moulding onto the person's leg.

10. Orthopaedic apparatus according to claim 8 or 9, characterised in that the lower supporting surface (15) clings to the upper part of the malleoli.

11. Orthopaedic apparatus according to one of the claims 1 to 10, characterised in that it comprises means for limiting the extension and/or flexion.

12. Orthopaedic apparatus according to one of the claims 1 to 11, characterised in that the thigh retention element (1) comprises an upper fixing member (37) comprising a front band (38) provided with a flexible lining of greater width than the loop (6), fixed to the ends of the said loop (6), the inner side end fixing (40), permitting a slight longitudinal displacement of the loop (6) in respect of the thigh and a lower fixing member comprising a front band (44) provided with a flexible lining the inner side thigh part of which comprises a sleeve (45) with axis substantially parallel to that of the thigh into which sleeve passes the inner upright (5) of the thigh retention element (1), the opening of the sleeve (45) having a width greater than that of the upright (5), such that this latter may be displaced slightly towards the front or towards the rear as well as longitudinally in respect of the thigh, the band (44) passing from the other side into a buckle (46) fixed on the outer face of the outer upright (4) the said bands (38) and (44) being extended at their ends by straps (42, 47) provided with clamping and fastening means.

13. Orthopaedic apparatus according to claim 12, characterised in that the front band (38) of the upper fixing member (37) comprises in its middle part a sleeve (41) into which the loop (6) passes, the width of the opening of the sleeve (41) being greater than that of the loop (6) in order that this latter may be slightly displaced there in respect of the thigh.

14. Orthopaedic apparatus according to claim 12 or 13, characterised in that the straps (42, 47) of the fixing members (37, 43) have a certain amount of elasticity with a view to improving the comfort.

15. Orthopaedic apparatus according to one of the claims 12 to 14, characterised in that the front band (44) of the lower fixing members (43) comprises a knee-cap clearance cut-out.

## Patentansprüche

1. Orthopädisches Gerät für ein instabiles Kniegelenk als Folge von Verletzungen an Sehnenbändern, an Gelenken, Nerven- oder Muskelsträngen, das ein Element (1) zur Aufrechterhaltung des Oberschenkels aufweist, welches aus einem äußeren Stützstab (4) und einem inneren Stützstab (5) besteht, die über eine Stützschlaufe (6) verbunden sind, wobei ein Element (2) zur Aufrechterhaltung des Beines vorgesehen ist, welches in gleicher Weise aus einem äußeren Stützstab (10) und einem inneren Stützstab (11) besteht, die über eine Stützschlaufe verbunden sind, und wobei die äußeren Stützstäbe (4, 10) und die inneren Stützstäbe (5, 11) über zwei Seitengelenke miteinander verbunden sind, **dadurch gekennzeichnet**, daß das äußere Gelenk aus zwei gelenkig verbundenen Schenkeln (18 und 21-23-24) besteht, um eine Ausdehnung nach vorne zu begrenzen, daß die Länge des einen Schenkels (18; 21-23-24) regulierbar ist und daß das innere Gelenk aus einem oberen Schenkel (25) besteht, der zur Gelenkverbindung mit dem oberen Teil eines Kreuzstückes (27) um eine im wesentlichen transversale Achse drehbar gelagert ist, wobei der untere Teil des Kreuzstückes an das Ende einer Stange (31) gelenkig angebracht ist und wobei das andere Ende der Stange mit dem unteren Schenkel (37) des besagten inneren Gelenkes zu einer Schließmechanismus-Achse verbunden ist und daß die Länge der Stange (31) regulierbar ist.

2. Orthopädisches Gerät nach Anspruch 1, **dadurch gekennzeichnet**, daß die Gelenke als Gabelbügel-Gelenke ausgebildet sind.

3. Orthopädisches Gerät nach Anspruch 1 oder 2, **dadurch gekennzeichnet**, daß die Schenkel (18; 21-23-24) regulierbarer Länge des äußeren Gelenkes und die Stange (31) des inneren Gelenkes aus zwei Teilen (33 und 34 oder 21 und 23-24) bestehen, von denen jeweils eines eine Gewindestange trägt, die in ein entsprechendes, mit einem Innengewinde versehenes Ende des jeweiligen anderen Teiles eingreift.

4. Orthopädisches Gerät nach einem der Ansprüche 1 bis 3, **dadurch gekennzeichnet**, daß auf den Gewindestangen der Schenkel (21-23-24) und der Stange (31) Sicherungs-Gegenmuttern (21', 35) befestigt sind, die die Rotation eines Ihrer Teile im Hinblick auf das andere Teil begrenzen oder blockieren.

5. Orthopädisches Gerät nach einem der Ansprüche 1 bis 4, **dadurch gekennzeichnet**, daß die Stützschlaufen des Elementes (1) zur Aufrechterhaltung des Oberschenkels und das Element (2) zur Aufrechterhaltung des Beines in ihrem mittleren Bereich gelenkig verbunden sind.

6. Orthopädisches Gerät nach einem der Ansprüche 1 bis 5, **dadurch gekennzeichnet**, daß das Element zur Aufrechterhaltung des Beines (2) aus zwei Stützschlaufen (12, 13) besteht.

7. Orthopädisches Gerät nach einem der Ansprüche 1 bis 6, **dadurch gekennzeichnet**, daß das Element zur Aufrechterhaltung des Oberschenkels (1) zwischen seiner Stützschlaufe (6) und seinen Stützstäben (4, 5) Füllstäbe (7) aufweist.

8. Orthopädisches Gerät nach einem der Ansprüche 1 bis 7, **dadurch gekennzeichnet**, daß an den inneren Flächen der Stützschlaufen (12, 13) Stützflächen (14, 15) aus einem flexiblen Material befestigt sind.

9. Orthopädisches Gerät nach einem der Ansprüche 1 bis 8, **dadurch gekennzeichnet**, daß die Stützflächen (14, 15) durch einen Abdruck an einem Bein einer betroffenen Person geformt sind.

10. Orthopädisches Gerät nach Anspruch 9, **dadurch gekennzeichnet**, daß die untere Stützfläche (15) an den oberen Bereich der Fußknöchel angeklammert ist.

11. Orthopädisches Gerät nach einem der Ansprüche 1 bis 10, **dadurch gekennzeichnet**, daß Mittel zur Begrenzung der Ausdehnung und des Durchbiegens vorgesehen sind.

12. Orthopädisches Gerät nach einem der Ansprüche 1 - 11, **dadurch gekennzeichnet**, daß das Element (1) zur Aufrechterhaltung des Oberschenkels aus einer oberen Befestigungsvorrichtung (37) besteht, die ein vorderes Band (38) mit einer flexiblen Ummantelung aufweist, die einen größeren Umfang als die Stützschlaufe (6) hat, und die am Ende der besagten Stützschlaufe befestigt ist, und daß die an der inneren Seite liegende Endbefestigung (40) eine einfache Längsverschiebung der Stützschlaufe (6) in Bezug auf den Oberschenkel ermöglicht, daß eine untere Befestigungsvorrichtung vorgesehen ist, die ein unteres Band (44) mit einer flexiblen Ummantelung umfaßt, die in einem Bereich an der inneren Seite des Oberschenkels eine Muffe (45) mit einer im wesentlichen zur Achse des Oberschenkels parallelen Achse aufweist, durch die die innere Stützstange (5) des Elementes (1) zur Aufrechterhaltung des Oberschenkels durchgeführt ist, daß die Öffnung der Muffe (45) einen größeren Umfang als die Stützstange (5) hat, so daß die Stützstange leicht nach vorne oder hinten und längs in Bezug auf den Oberschenkel verschoben werden kann, daß das Band (44) auf der anderen Seite in einer Schlaufe (46) geführt wird, die an der äußeren Seite der äußeren Stützstange (4) befestigt ist, und daß die besagten Bänder (38) und (44) an ihren Enden durch Tragriemen verlängert sind, die Mittel zum Klemmen oder Befestigen aufweisen.

13. Orthopädisches Gerät nach Anspruch 12, **dadurch gekennzeichnet**, daß das vordere Band (38) der oberen Befestigungsvorrichtung (37) in seiner Mitte eine Muffe (41) aufweist, in welcher die Stützschlaufe (6) geführt wird, wobei die Weite der Öffnung größer ist, als die der Stützschlaufe (6), um diese leicht am Oberschenkel verschieben zu können.

14. Orthopädisches Gerät nach Anspruch 12 oder 13, **dadurch gekennzeichnet**, daß die Bänder (42, 47) der Befestigungsmittel (37, 43) zur Erhöhung des Komforts eine gewisse Elastizität aufweisen.

15. Orthopädisches Gerät nach einem der Ansprüche 12 bis 14, **dadurch gekennzeichnet**, daß das vordere Band (44) des Befestigungsmittels (43) eine bogenförmige Aussparung für das Kniestück aufweist.

## FIG.1

## FIG. 2

FIG. 3

FIG. 4